(19) Europäisches Patentamt
European Patent Office
Office européen des brevets

(11) EP 4 738 370 A1

(12) EUROPEAN PATENT APPLICATION
published in accordance with Art. 153(4) EPC

(43) Date of publication:
06.05.2026 Bulletin 2026/19

(21) Application number: 24831498.1

(22) Date of filing: 22.05.2024

(51) International Patent Classification (IPC):
G16B 30/00 (2019.01)

(52) Cooperative Patent Classification (CPC):
G16B 30/00

(86) International application number:
PCT/JP2024/018842

(87) International publication number:
WO 2025/004627 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.06.2023 JP 2023108941

(71) Applicant: ARKRAY, Inc.
Kyoto-shi, Kyoto 601-8045 (JP)

(72) Inventor: UCHIYAMA, Makoto
Kyoto-shi, Kyoto 602-0008 (JP)

(74) Representative: MFG Patentanwälte
Meyer-Wildhagen Meggle-Freund
Gerhard PartG mbB
Amalienstraße 62
80799 München (DE)

Remarks:
The complete document including Reference
Table(s) and the Sequence Listing(s) can be
downloaded from the EPO website

(54) CLASSIFICATION METHOD, CLASSIFICATION DEVICE, CLASSIFICATION SYSTEM, CLASSIFICATION PROGRAM, AND RECORDING MEDIUM

(57) There is provided a classification method including: measuring a base sequence of a nucleic acid molecule in a measurement sample, as a measurement sequence; and classifying the measurement sequence according to a plurality of groups, into each of which base sequences of a plurality of nucleic acid molecules are grouped according to a specific rule, based on a similarity between a representative sequence and the measurement sequence that has been measured in the measuring, each of the representative sequences being set as a base sequence representing one of the respective groups.

FIG.1

**Description**

Technical Field

The present disclosure relates to a classification method, a classification device, a classification system, a classification program, and a recording medium.

Background Art

In recent years, comprehensive quantitative analysis of nucleic acids is represented by next-generation sequencing (hereinafter, may be abbreviated as NGS), and has been applied to determination of diseases including cancer.

With development of comprehensive analysis technologies, the number of analyzable molecular species has dramatically increased. However, as the number of molecular species to be analyzed increases, a calculation amount required for analysis explosively increases. As a result, serious problems, such as an increase in facility cost and a decrease in throughput due to the necessity of high-performance computing devices and long-time analysis, often occur.

In the related art, in order to prevent such an increase in calculation amount, there has been proposed a technology of reducing the amount of calculation by reducing the number of molecules to be analyzed through selection of important molecules using statistical analysis or machine learning (refer to Non-Patent Documents 1 and 2).

Non-Patent Document 1: Jin_et_al, 2017, Clinical Cancer Research, Evaluation of Tumor-Derived Exosomal miRNA as Potential Diagnostic Biomarkers for Early-Stage Non-Small Cell Lung Cancer Using Next-Generation Sequencing

Non-Patent Document 2: Asakura_et_al, 2020, Communications Biology, A miRNA-based diagnostic model predicts resectable lung cancer in humans with high accuracy

SUMMARY OF INVENTION

Technical Problem

In an analysis process using comprehensive molecular species analysis, the following two steps are exemplified as processes with a large processing amount (specifically, calculation amount).

    Step 1: Classification of target nucleic acid molecules
    Step 2: Disease determination/prediction

Here, for example, approximately 2600 types of known microRNAs exist in microRNAs in human blood, and several millions of microRNAs exist in a few $\mu$L of a blood sample. In addition, in a case of performing the above-described step 1 on several millions of microRNAs, for example, processing of comparing the several millions of microRNAs with each of approximately 2600 known microRNAs and classifying the several millions of microRNAs is performed. As a result, the processing amount (specifically, the calculation amount) is enormous.

In the method of selecting important molecules using statistical analysis or machine learning (refer to Non-Patent Documents 1 and 2), processing of comprehensively detecting molecular species and then reducing the number of molecular species to be analyzed is performed. As a result, the processing amount (specifically, the calculation amount) in subsequent machine learning or statistical analysis is reduced, and thus, the processing amount in the above-described step 2 can be reduced.

However, in the methods of Non-Patent Documents 1 and 2, the processing amount in the above-described step 1 cannot be reduced, and there still remain problems related to an increase in facility cost and a decrease in throughput due to the necessity of high-performance computing devices and long-time analysis.

An object of the present disclosure is to provide a classification method, a classification device, a classification system, a classification program, and a recording medium capable of reducing a processing amount when classifying a base sequence of a nucleic acid molecule. Solution to Problem

According to an aspect of the present disclosure, there is provided a classification method including: measuring a base sequence of a nucleic acid molecule in a measurement sample, as a measurement sequence; and classifying the measurement sequence according to a plurality of groups, into each of which base sequences of a plurality of nucleic acid molecules are grouped according to a specific rule, based on a similarity between a representative sequence and the measurement sequence that has been measured in the measuring, each of the representative sequences being set as a base sequence representing one of the respective groups. Advantageous Effects of Invention

According to the present disclosure, it is possible to reduce a processing amount when classifying a base sequence of a nucleic acid molecule.

BRIEF DESCRIPTION OF DRAWINGS

Fig. 1 is a flowchart illustrating an example of each step of a classification method according to the present embodiment.
Fig. 2 is a block diagram illustrating an example of a computer that functions as a classification device according to the present embodiment.
Fig. 3 is a block diagram illustrating an example of a functional configuration of the classification device according to the present embodiment.
Fig. 4 is a list showing 56 types of microRNAs to be classified in the present example.
Fig. 5 is a list showing each group obtained by grouping the 56 types of microRNAs shown in Fig. 4 and a representative sequence that is set for each group.

DESCRIPTION OF EMBODIMENTS

Hereinafter, an example of an embodiment of a technology according to the present disclosure will be described with reference to the drawings. Note that components and processes having the same operations, actions, and functions are denoted by the same reference numerals throughout the drawings, and redundant description may be omitted as appropriate. Each drawing is only schematically illustrated to an extent that the technology according to the present disclosure can be sufficiently understood. Therefore, the technology according to the present disclosure is not limited only to the illustrated example. Further, in the present embodiment, description of configurations that are not directly related to the present disclosure or well-known configurations may be omitted.

<Classification Method 10>

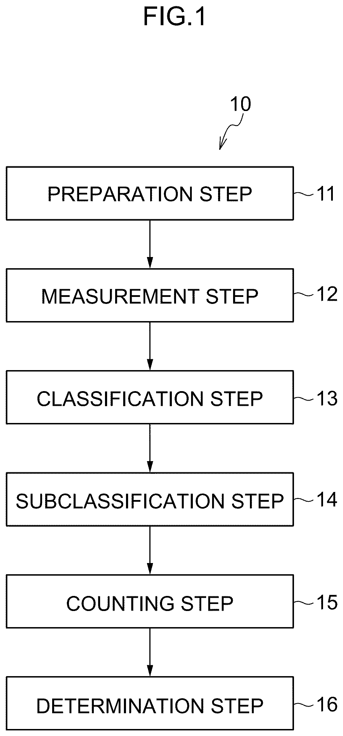

First, a classification method 10 according to the present embodiment will be described. Fig. 1 is a schematic diagram illustrating an example of each step of a classification method 10 according to the present embodiment.
The classification method 10 is a method of measuring a base sequence of a nucleic acid molecule in a measurement sample and classifying the base sequence according to the groups. Examples of the measurement sample include body fluids (for example, blood, serum, urine, tears, saliva, sweat, semen, lymph, tissue fluid, body cavity fluid (for example, effusion fluid, ascites, and the like), cerebrospinal fluid, amniotic fluid, vaginal fluid, nasal discharge, and the like), tissues, and cells. A subject from which the measurement sample is collected may be a human or a non-human animal. Examples of the non-human animal include non-human mammals (monkey, dog, cat, mouse, rat, rabbit, cow, horse, pig, sheep, and the like) and birds (chicken, quail, and the like).
Examples of the nucleic acid molecule include microRNAs. Note that the nucleic acid molecule may be a small RNA other than the microRNA, another RNA, a DNA, or the like. Further, the nucleic acid molecule is not necessarily a nucleic acid molecule including only bases of ATGCU. Specifically, examples of the nucleic acid molecule include nucleic acids subjected to modification such as DNA/RNA methylation and editing such as A-to-I RNA editing. As described above, the present classification method can be applied to various nucleic acid molecules.
Specifically, as illustrated in Fig. 1, the classification method 10 includes a preparation step 11, a measurement step 12, a classification step 13, a subclassification step 14, a counting step 15, and a determination step 16. In the present embodiment, the preparation step 11, the measurement step 12, the classification step 13, the subclassification step 14, the counting step 15, and the determination step 16 are executed in this order as an example.
Note that, since the classification method 10 is a method including the counting step 15 and the determination step 16, the classification method is also referred to as a counting method or a determination method. In addition, in a case where inspection, analysis, or the like is performed by determination in the determination step 16, the classification method is also referred to as an inspection method or an analysis method. Hereinafter, each step of the classification method 10 will be described.

<Preparation Step 11>

The preparation step 11 is a step executed as preparation for executing the classification step 13. In the present embodiment, the preparation step 11 is performed, for example, before the measurement step 12 and the classification step 13 are performed. Note that the preparation step 11 may be executed at least before the classification step 13 is executed, and may be executed after the measurement step 12.

In the preparation step 11, base sequences of a plurality of nucleic acid molecules are grouped into a plurality of groups according to a specific rule, and each of the representative sequences is set as a base sequence representing one of the respective groups. Hereinafter, among the base sequences included in each of the plurality of groups, base sequences other than the representative sequence may be referred to as similar sequences.

Each of the plurality of groups grouped in the preparation step 11 may include a representative sequence and one or more similar sequences. That is, the representative sequence and the one or more similar sequences belong to each of the plurality of groups. Note that the group may include only the representative sequence. That is, the group may be a group that does not include similar sequences.

Here, for example, approximately 2600 types of known microRNAs exist in microRNAs in human blood. In a case where base sequences of microRNAs in human blood are to be classified, the base sequences of approximately 2600 types of microRNAs are grouped.

As the representative sequence, for example, the longest base sequence among the base sequences included in the group may be set. Note that the representative sequence is not limited to the longest base sequence among the base sequences included in the group, and for example, a base sequence having a highest expression level among the base sequences included in the group may be set. The base sequence having the highest expression level can be set by, for example, the following first method or second method.

Here, the base sequence of the nucleic acid molecule that is present in the measurement sample and has a large amount is determined for each measurement sample. That is, for example, in the case of human blood, a microRNA that exists in human blood and has a large amount is determined. The first method is a method of setting in advance, using the fact, the base sequence of the nucleic acid molecule that is present in the measurement sample and has the largest amount among the base sequences included in the group, as the representative sequence indicating the base sequence having a highest expression level. Specifically, in the first method, for example, based on measurement data obtained by measuring a plurality of measurement samples which are samples for setting, statistical data, or the like, the base sequence of the nucleic acid molecule that is present in the measurement sample and has the largest amount among the base sequences included in the group can be set in advance as the representative sequence indicating the base sequence having a highest expression level.

The second method is a method of setting, based on a measurement result measured in the measurement step 12, the base sequence having a highest expression level among the base sequences included in the group as the representative sequence. In the second method, for example, in the measurement step 12, the base sequence having a largest measured amount among the base sequences included in the group can be set as a representative sequence indicating the base sequence having a highest expression level. Therefore, in the second method, the representative sequence may be changed depending on the measurement result of the measurement step 12. Further, in the second method, the preparation step 11 is performed after the measurement step 12.

In addition, the representative sequence may be a sequence that is artificially defined instead of a sequence that exists in nature. Examples of the sequence include a sequence that is partially amplified by polymerase chain reaction (PCR) or the like.

The specific rule is based on a similarity of the base sequences grouped into the plurality of groups (specifically, the one or more similar sequences) to the representative sequence or a similarity between the base sequences grouped into the plurality of groups (specifically, the representative sequence and the one or more similar sequences).

For example, in a case where the similarity of the one or more similar sequences to the representative sequence is set as a specific rule, it is possible to set a specific rule that a difference of the similar sequence to the representative sequence is a difference of n bases or fewer (n= natural number). In this case, the specific rule may include a similarity of the representative sequence to a reverse complementary strand of the representative sequence. That is, it is possible to set a specific rule that a difference from the representative sequence or the reverse complementary strand of the representative sequence is n bases or fewer (n= natural number).

In addition, in a case where a similarity between the base sequences (specifically, the representative sequence and the one or more similar sequences) grouped into the plurality of groups is set as a specific rule, it is possible to set a specific rule that a difference between the base sequences is a difference of n bases or fewer (n= natural number). In this case, the specific rule may also include a similarity of the base sequence to a reverse complementary strand of the base sequence. That is, it is possible to set a specific rule that a difference between the base sequences or a difference of the base sequence from a reverse complementary strand of the base sequence is n bases or fewer (n= natural number).

The above-described difference includes, for example, base substitutions, more bases, fewer bases, base additions, and base deletions. As the similarity, for example, a similarity score used in homology search of base sequences can be used.

When determining the similarity, it may be also possible to focus on sequences on the 5' and 3' sides in the base sequences (that is, terminal sequences). That is, for example, the base sequences may be grouped by a similarity (specifically, a difference) between the sequences on the 5' and 3' sides in the base sequences (that is, terminal sequences).

Note that the specific rule is not limited to a rule based on the similarity of the one or more similar sequences to the representative sequence and a rule based on the similarity between the base sequences (specifically, the representative sequence and the one or more similar sequences) grouped into the plurality of groups.

For example, the base sequences may be grouped according to a specific rule that the base sequences include a specific motif (for example, a specific base sequence or a consensus sequence). Specifically, for example, the base sequences including a specific base sequence (refer to the following sequence) can be grouped as one group.

AT** A *C* A *************(* may be any ATGCU)

In addition, for example, a base sequence of a precursor, an isoform base sequence, and a base sequence that serves as a basis of these base sequences may be grouped as one group.

Further, grouping may be performed by combining a plurality of specific rules for the above-described grouping.

In the present embodiment, the preparation step 11 is executed, for example, when the classification method is executed first. When the classification method is executed for the second and subsequent times, the classification step 13 can be executed using the groups obtained by grouping and the representative sequence that is set, the groups and the representative sequence being obtained when the classification method is executed first.

Therefore, the preparation step 11 does not need to be performed every time the classification method is performed. Note that, in a case where grouping of groups and setting of a representative sequence are changed, the preparation step 11 may be executed once in a plurality of times or every time when the classification method is executed for the second and subsequent times.

<Measurement Step 12>

The measurement step 12 is a step of measuring the base sequence of the nucleic acid molecule in the measurement sample as a measurement sequence. Specifically, in the measurement step 12, the base sequence of the nucleic acid molecule in the measurement sample is measured as a measurement sequence by using next-generation sequencing (NGS) that is a measurement device.

<Classification Step 13>

The classification step 13 is a step of classifying the measurement sequences according to the groups based on the similarity between each of the representative sequences being set as a base sequence representing one of the respective groups and the measurement sequence measured in the measurement step 12. That is, in the classification step 13, the measurement sequences are compared with the representative sequence by the similarity, and the measurement sequences are classified according to the groups.

In the classification step 13, for example, the measurement sequences are classified into a group having a highest similarity between the representative sequence and the measurement sequence. Specifically, in the classification step 13, for example, the measurement sequences are classified into a group having a highest similarity score, the similarity score being used in homology search of base sequences. In this case, the measurement sequences are classified into one group. In the classification step 13, all the measurement sequences measured in the measurement step 12 are classified into groups.

In the classification step 13, the measurement sequences may be classified into a group in which the similarity between the representative sequence and the measurement sequence is equal to or higher than a threshold value. In this case, the measurement sequences may be classified into a plurality of groups. As described above, in the classification step 13, the measurement sequences may be classified into a plurality of groups. That is, it is not necessary that the measurement sequence and the group have a one-to-one correspondence.

The measurement sequence does not need to be the same as any of the base sequences which are grouped. In addition, the measurement sequence that is classified for each group may be compared with the measurement sequence in another group, and may be classified again before counting.

<Subclassification Step 14>

The subclassification step 14 is a step of subclassifying the measurement sequences which are classified into each of the plurality of groups into each of the base sequences included in the group after the classification step 13. That is, the measurement sequences which are classified into the groups are further classified into any one of the representative sequence and the one or more similar sequences belonging to the group.

In the subclassification step 14, for example, the measurement sequences are classified into a sequence having a highest similarity between any one of the representative sequence and the one or more similar sequences and the measurement sequence. Specifically, in the subclassification step 14, for example, the measurement sequences are subclassified into any one of the representative sequence having a highest similarity score and the one or more similar sequences, the similarity score being used in homology search of base sequences.

Note that the execution of the subclassification step 14 is not essential, and the counting step 15 may be executed without executing the subclassification step 14 after the classification step 13.

<Counting Step 15>

The counting step 15 is a step of counting the number of molecules of the measurement sequences which are classified into each of the plurality of groups in the classification step 13. Specifically, in the counting step 15, the number of molecules of the measurement sequences which are subclassified into any one of the representative sequence and the one or more similar sequences in the subclassification step 14 is counted for each of the base sequences (that is, the representative sequence and the one or more similar sequences).

Note that, in a case where the counting step 15 is performed without performing the subclassification step 14 after the classification step 13, in the counting step 15, the number of molecules of the measurement sequences which are classified into each of the plurality of groups is counted for each group.

<Determination Step 16>

The determination step 16 is a step of performing specific determination on the measurement sample based on the number of molecules that is counted by the counting step 15.

The specific determination includes disease determination/prediction of a provider who provided the sample. The disease determination/prediction can be performed, for example, by a distribution of the number of molecules (that is, the expression levels of the nucleic acids) of the measurement sequences counted for each of the base sequences (that is, the representative sequence and the one or more similar sequences) in the counting step 15.

Specifically, for example, a disease or the like is determined using the expression levels of the microRNAs by an arbitrary determination formula in which the number of molecules of each group is set as a variable. For example, the following determination formula can be used.

Determination formula $= Y_1 \times$ expression level of group 1 + ... + $Y_n \times$ expression level of group n + X (Y is a coefficient determined in advance for each group, and X is a constant determined in advance)

Note that a disease or the like may be determined using the expression levels of the microRNAs by machine learning. Specifically, machine learning is performed in advance by using, as training data, the expression level of each of the target microRNAs and information on the presence or absence of a disease. Thus, a learning model is constructed. Then, in a case where the measurement sequences counted in the counting step 15 and the expression levels of the measurement sequences are input to the learning model as input factors, the presence or absence of a disease that is an output factor can be determined by the learning model.

In addition, the determination step 16 is not limited to the disease determination/prediction, and basic biological analysis, regression prediction, abnormality detection, or the like may be performed.

<Classification System 20>

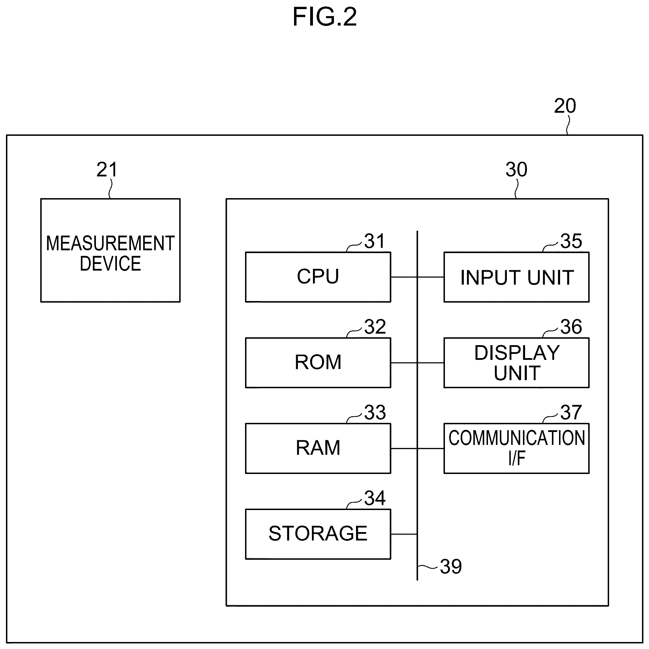

Next, a classification system 20 as a system that executes the above-described classification method will be described. As illustrated in Fig. 2, the classification system 20 includes a measurement device 21 and a classification device 30.

<Measurement Device 21>

The measurement device 21 is an example of a measurement unit, and is a device that executes the above-described measurement step 12. That is, the measurement device 21 measures the base sequence of the nucleic acid molecule in the measurement sample as a measurement sequence. As the measurement device 21, for example, NGS is used.

<Classification Device 30>

The classification device 30 is an example of a classifier, and is a device that executes the above-described classification step 13. That is, the classification device 30 classifies the measurement sequences according to the groups based on the similarity between the representative sequence and the measurement sequence measured by the measurement device 21, each of the representative sequences being set as a base sequence representing one of the respective groups into which the base sequences of the plurality of nucleic acid molecules are grouped according to a specific rule. Further, the classification device 30 executes the above-described subclassification step 14, the counting step 15, and the determination step 16.

The classification device 30 has a function as a computer, and as illustrated in Fig. 2, includes a central processing unit (CPU) 31, a read only memory (ROM) 32, a random access memory (RAM) 33, a storage 34, an input unit 35, a display unit 36, and a communication interface (I/F) 37. These units are communicably connected to each other via a bus 39.

The CPU 31 is a central processing unit, and executes various programs and controls each unit. That is, the CPU 31 reads the program from the ROM 32 or the storage 34, and executes the program by using the RAM 33 as a work area. The CPU 31 performs control of each of the units and various types of arithmetic processing according to the program stored in the ROM 32 or the storage 34. Note that the CPU 31 is an example of a processor.

The ROM 32 records various programs and various data. The RAM 33 temporarily stores programs or data by using a work area. The storage 34 includes a hard disk drive (HDD) or a solid state drive (SSD), and records various programs including an operating system and various data.

In the present embodiment, for example, a classification program causing the classification device to execute classification processing for performing the above-described classification method is recorded in the storage 34. The classification program may be one program or a program group including a plurality of programs or modules.

In addition, in the storage 34, group information of each of the groups that are grouped and sequence information of each of the representative sequences that is set as a base sequence representing one of the respective groups are recorded, the group information and the sequence information being obtained in the above-described preparation step 11. Note that the classification program, the group information, and the sequence information may be recorded in the ROM 32. The ROM 32 or the storage 34 functions as an example of a non-transitory recording medium.

The input unit 35 includes a pointing device such as a mouse and a keyboard, and is used to receive various inputs. In addition, the input unit 35 receives, as an input, information of the measurement sequence that is measured by the measurement device 21.

The display unit 36 is, for example, a liquid crystal display, and displays various types of information. The display unit 36 may function as the input unit 35 by adopting a touch panel system.

The communication interface 37 is an interface for performing communication with other devices, and for example, standards such as Ethernet (registered trademark), Fiber Distributed Data Interface (FDDI), and Wi-Fi (registered trademark) are used.

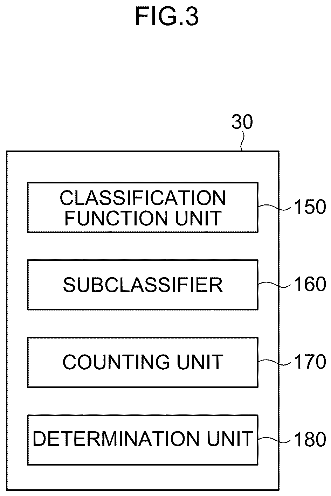

As illustrated in Fig. 3, in a case where the CPU 31 of the classification device 30 executes the classification program, the CPU 31 functions as a classification function unit 150, a subclassifier 160, a counting unit 170, and a determination unit 180.

The classification function unit 150 executes the above-described classification step 13. That is, the classification function unit 150 classifies the measurement sequences according to the groups based on the similarity between the representative sequence and the measurement sequence measured by the measurement device 21, the representative sequence being set as a base sequence representing one of the respective groups into which the base sequences of the plurality of nucleic acid molecules are grouped according to a specific rule (refer to the above-described classification step 13).

The subclassifier 160 executes the above-described subclassification step 14. That is, the subclassifier 160 subclassifies the measurement sequences which are classified into each of the plurality of groups by the classification function unit 150 into each of the base sequences included in the group (refer to the above-described subclassification step 14).

The counting unit 170 executes the counting step 15. That is, the counting unit 170 counts the number of molecules of the measurement sequences that are classified into each of the plurality of groups in the classification function unit 150. Specifically, the counting unit 170 counts the number of molecules of the measurement sequences that are subclassified into any one of the representative sequence or the one or more similar sequences by the subclassifier 160 for each of the base sequences (that is, the representative sequence and the one or more similar sequences) (refer to the above-described counting step 15).

The determination unit 180 executes the above-described determination step 16. That is, the determination unit 180 performs specific determination on the measurement sample based on the number of molecules that are counted by the counting unit 170 (refer to the above-described determination step 16).

Note that, in the present embodiment, the classification system 20 includes the measurement device 21 and the classification device 30, but the classification system 20 may be configured by one device. In this case, the one device functions as an example of the measurement unit and the classifier.

In addition, the classification device 30 may include a plurality of devices. For example, the classification device 30 may include a plurality of (for example, four) devices that share and execute the above-described classification step 13, the subclassification step 14, the counting step 15, and the determination step 16.

<Example>

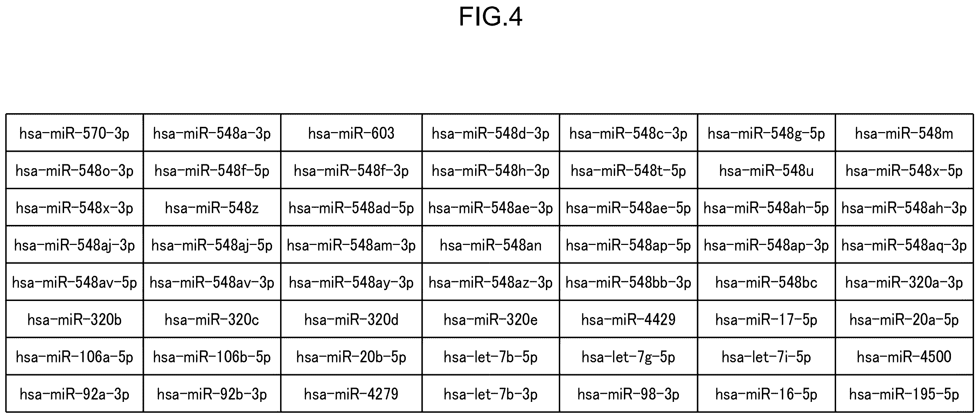

Next, as an example, an example in which blood including microRNAs (an example of nucleic acid molecules) is used as a measurement sample and each step of the above-described classification method is executed will be described. In the present example, for convenience, each step of the above-described classification method is executed for 56 types of microRNAs (refer to Fig. 4).

Note that the example illustrates an example of the technology according to the present disclosure, and the technology according to the present disclosure is not limited to the content of the example.

<Preparation Step 11>

In the present example, 56 types of microRNAs were grouped according to a specific rule that the difference is 3 bases or fewer from a base sequence which is to be the representative sequence or a reverse complementary strand of the base sequence. As the representative sequence, the longest base sequence in the group was set. Thus, the similar sequences differ by 3 bases or fewer in the same sequence as compared to the longest representative sequence. The difference includes, for example, base substitutions, more bases, fewer bases, base additions, and base deletions with respect to the representative sequence.

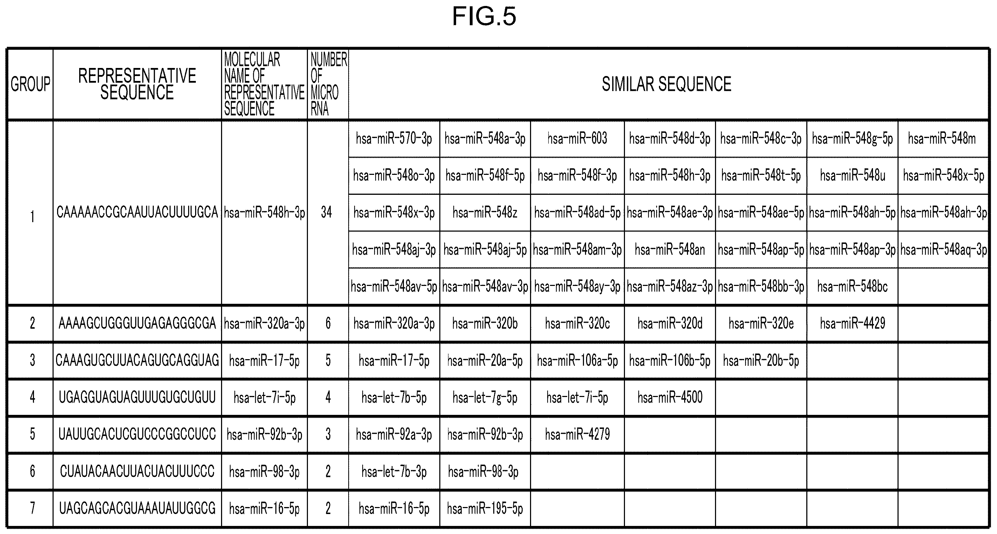

In the present example, 56 types of microRNAs (refer to Fig. 4) were grouped, and representative sequences were set as shown in Fig. 5 based on the specific rule.

The representative sequences that are set are as follows.

CAAAAACCGCAAUUACUUUUGCA (Sequence Number 1) (hsa-miR-548h-3p)
AAAAGCUGGGUUGAGAGGGCGA (Sequence Number 2) (hsa-miR-320a-3p)
CAAAGUGCUUACAGUGCAGGUAG (Sequence Number 3) (hsa-miR-17-5p)
UGAGGUAGUAGUUUGUGCUGUU (Sequence Number 4) (hsa-let-7i-5p)
UAUUGCACUCGUCCCGGCCUCC (Sequence Number 5) (hsa-miR-92b-3p)
CUAUACAACUUACUACUUUCCC (Sequence Number 6) (hsa-miR-98-3p)
UAGCAGCACGUAAAUAUUGGCG (Sequence Number 7) (hsa-miR-16-5p)

<Measurement Step 12>

In the present example, a base sequence of a microRNA in blood that is a measurement sample is measured as a measurement sequence by NGS that is a measurement device, and the number of measurement sequences is quantified.

<Classification Step 13>

In the present example, all the measurement sequences measured in the measurement step 12 are classified into a group having a highest similarity score, the similarity score indicating a similarity with the representative sequence of each of the plurality of groups. Note that microRNAs other than the 56 types of microRNAs were excluded in advance from targets to be classified.

<Subclassification Step 14>

In the present example, after the classification step 13, all the measurement sequences which are classified into each of the plurality of groups are subclassified into each of the representative sequence and the one or more similar sequences in the group. In this case, the measurement sequences are subclassified into any one of the representative sequence having a highest similarity score and the one or more similar sequences, the similarity score indicating a similarity with the measurement sequence.

<Counting Step 15>

In the present example, the number of molecules of the measurement sequences which are subclassified into any one of the representative sequence and the one or more similar sequences in the subclassification step 14 is counted for each of the base sequences (the representative sequence and the one or more similar sequences).

<Determination Step 16>

In the present example, in the counting step 15, disease determination/prediction is performed by the distribution of the number of molecules (that is, the expression levels of the nucleic acids) of the measurement sequences which are counted for each of the base sequences (the representative sequence and the one or more similar sequences).

Specifically, for example, a disease or the like is determined using the expression levels of the microRNAs by the following determination formula in which the number of molecules of each group is set as a variable.

Determination formula = $Y_1 \times$ expression level of group 1 + $\cdots$ + $Y_7 \times$ expression level of group 7 + X (Y is a coefficient determined in advance for each group, and X is a constant determined in advance)

Note that a disease or the like may be determined using the expression levels of the microRNAs by machine learning. Specifically, machine learning is performed in advance by using, as training data, the expression level of each of 56 types of target microRNAs and information on the presence or absence of a disease, and thus, a learning model is constructed. In addition, in a case where the learning model receives, as input factors, the measurement sequences which are counted in the counting step 15 and the expression levels of the measurement sequences, the learning model can determine, as an output factor, the presence or absence of a disease.

<Effects of Example>

According to the present example, as will be described later, in the classification step 13, the processing amount (specifically, calculation amount) when classifying the base sequences of the microRNAs can be reduced.

For example, in a case where the number of measurement sequences is 10,000,000 and the calculation amount required for comparing one measurement sequence with one base sequence is A, when the measurement sequences are classified into each of 56 types of microRNAs, the following calculation amount is obtained.

$$10,000,000 \text{ (molecules)} \times 56 \text{ (types)} = 560,000,000 \text{ ways} \cdots (1)$$

$$\text{Based on (1), total calculation amount} = 560,000,000 \times A \cdots (2)$$

On the other hand, in the present example, 56 types of microRNAs are grouped into 7 types of groups, and the measurement sequences are classified based on the similarity with the representative sequence. Thus, the following calculation amount is obtained.

$$10,000,000 \text{ (molecules)} \times 7 \text{ (types)} = 70,000,000 \text{ ways} \cdots (3)$$

$$\text{Based on (3), total calculation amount} = 70,000,000 \times A \cdots (4)$$

$$\text{Based on (2) and (4), } 70,000,000 \times A/560,000,000 \times A = 0.125 \cdots (5)$$

Based on (5), according to the present example, it can be seen that the calculation amount can be reduced to 0.125 times as compared with the case where the measurement sequences are classified into each of 56 types of microRNAs.

In a case where the subclassification step 14 is executed, the following calculation amount is required. Here, it is assumed that 2,000,000 (molecules) are virtually classified into each of groups 1, 2, and 3, and 1,000,000 (molecules) are virtually classified into each of groups 4, 5, 6, and 7.

Group 1: total calculation amount = 2,000,000 (molecules) $\times$ 34 (types) $\times$ A = 68,000,000     (11)

Group 2: total calculation amount = 2,000,000 (molecules) $\times$ 6 (types) $\times$ A = 12,000,000     (12)

Group 3: total calculation amount = 2,000,000 (molecules) $\times$ 5 (types) $\times$ A = 10,000,000     (13)

Group 4: total calculation amount = 1,000,000 (molecules) × 4 (types) × A = 4,000,000 ⋯ (14)

Group 5: total calculation amount = 1,000,000 (molecules) × 3 (types) × A = 3,000,000 ⋯ (15)

Group 6: total calculation amount = 1,000,000 (molecules) × 2 (types) × A = 2,000,000 ⋯ (16)

Group 7: total calculation amount = 1,000,000 (molecules) × 2 (types) × A = 2,000,000 ⋯ (17)

The total calculation amount based on the sum of (11) to (17) is as follows.

$$\text{Total calculation amount} = 111{,}000{,}000 \times A \cdots (18)$$

The sum of (4) and (18) (refer to (19) below) is the calculation amount in the classification step 13 and the subclassification step 14.

$$70{,}000{,}000 \times A + 111{,}000{,}000 \times A = 181{,}000{,}000 \times A \cdots (19)$$

$$\text{Based on (2) and (19),} \quad 181{,}000{,}000 \times A / 560{,}000{,}000 \times A = 0.323 \cdots (20)$$

Based on (20), according to the present example, it can be seen that the calculation amount can be reduced to 0.323 times as compared with the case where the present example is not used.

As described above, the technology according to the present disclosure has a technical idea of reducing the processing amount (specifically, the calculation amount) by collecting and grouping similar base sequences based on a fact that similar base sequences exist in nucleic acids.

<Appendixes>

(Aspect 1)

A classification method, including:

measuring a base sequence of a nucleic acid molecule in a measurement sample, as a measurement sequence; and
classifying the measurement sequence according to a plurality of groups, into each of which base sequences of a plurality of nucleic acid molecules are grouped according to a specific rule, based on a similarity between a representative sequence and the measurement sequence that has been measured in the measuring, each of the representative sequences being set as a base sequence representing one of the respective groups.

(Aspect 2)

The classification method according to Aspect 1, further including counting a number of molecules of the measurement sequence that has been classified according to the respective groups.

(Aspect 3)

The classification method according to Aspect 2, further including:

after classifying the measurement sequence, subclassifying the measurement sequence, which has been classified according to the respective groups, according to the respective base sequences included in the groups,

in which, in counting the number of molecules of the measurement sequence, the number of molecules of the measurement sequence that has been subclassified according to the base sequences is counted for each of the base sequences.

(Aspect 4)

The classification method according to Aspect 2 or Aspect 3, further including performing specific determination of the measurement sample based on the number of molecules counted in the counting.

(Aspect 5)

The classification method according to any one of Aspects 1 to 4, in which the specific rule is based on a similarity of each of the base sequences grouped into the plurality of groups to the representative sequences or a similarity between the base sequences grouped into the plurality of groups.

(Aspect 6)

The classification method according to any one of Aspects 1 to 5, in which the representative sequences are set as a longest base sequence among the base sequences included in the groups.

(Aspect 7)

The classification method according to any one of Aspects 1 to 6, in which the representative sequences are set as a base sequence having a highest expression level among the base sequences included in the groups.

(Aspect 8)

The classification method according to any one of Aspects 1 to 7, in which, in the classifying, the measurement sequence is classified as belonging to a group in which the similarity between the representative sequence and the measurement sequence is equal to or higher than a threshold value or a group in which the similarity between the representative sequence and the measurement sequence is highest.

(Aspect 9)

The classification method according to any one of Aspects 1 to 8, in which, in the measuring, a base sequence of a nucleic acid molecule in the measurement sample is measured as a measurement sequence using next-generation sequencing.

(Aspect 10)

A classification device including a processor, in which the processor is configured to classify a measurement sequence, which has been measured, according to a plurality of groups into each of which base sequences of a plurality of nucleic acid molecules are grouped according to a specific rule, based on a similarity between a representative sequence and the measurement sequence, each of the representative sequences being set as a base sequence representing one of the respective groups.

(Aspect 11)

A classification system including:

a measurement unit that measures a base sequence of a nucleic acid molecule in a measurement sample as a measurement sequence; and

a classifier that classifies the measurement sequence according to a plurality of groups into each of which base sequences of a plurality of nucleic acid molecules are grouped according to a specific rule, based on a similarity between a representative sequence and the measurement sequence that has been measured in the measurement unit, each of the representative sequences being set as a base sequence representing one of the respective groups.

(Aspect 12)

A classification program executable by a computer to perform classification processing comprising classifying a measurement sequence, which has been measured, according to a plurality of groups into each of which base sequences of a plurality of nucleic acid molecules are grouped according to a specific rule, based on a similarity between a representative sequence and the measurement sequence, each of the representative sequences being set as a base sequence representing one of the respective groups.

(Aspect 13)

A non-transitory recording medium storing a classification program, the classification program being executable by a computer to perform classification processing comprising classifying a measurement sequence, which has been measured, according to a plurality of groups into each of which base sequences of a plurality of nucleic acid molecules are grouped according to a specific rule, based on a similarity between a representative sequence and the measurement sequence, each of the representative sequences being set as a base sequence representing one of the respective groups.
The disclosure of Japanese Patent Application No. 2023-108941 filed on June 30, 2023 is incorporated herein by reference in its entirety. All documents, patent applications, and technical standards described in this specification are incorporated herein by reference to the same extent as if each individual document, patent application, or technical standard is specifically and individually indicated to be incorporated by reference.

**Claims**

1. A classification method, comprising:

   measuring a base sequence of a nucleic acid molecule in a measurement sample, as a measurement sequence; and
   classifying the measurement sequence according to a plurality of groups, into each of which base sequences of a plurality of nucleic acid molecules are grouped according to a specific rule, based on a similarity between a representative sequence and the measurement sequence that has been measured in the measuring, each of the representative sequences being set as a base sequence representing one of the respective groups.

2. The classification method according to Claim 1, further comprising counting a number of molecules of the measurement sequence that has been classified according to the respective groups.

3. The classification method according to Claim 2, further comprising:

   after classifying the measurement sequence, subclassifying the measurement sequence, which has been classified according to the respective groups, according to the respective base sequences included in the groups, wherein, in counting the number of molecules of the measurement sequence, the number of molecules of the measurement sequence that has been subclassified according to the base sequences is counted for each of the base sequences.

4. The classification method according to Claim 2, further comprising performing specific determination of the measurement sample based on the number of molecules counted in the counting.

5. The classification method according to Claim 1, wherein the specific rule is based on a similarity of each of the base sequences grouped into the plurality of groups to the representative sequences or a similarity between the base sequences grouped into the plurality of groups.

6. The classification method according to Claim 1, wherein the representative sequences are set as a longest base sequence among the base sequences included in the groups.

7. The classification method according to Claim 1, wherein the representative sequences are set as a base sequence having a highest expression level among the base sequences included in the groups.

8. The classification method according to Claim 1, wherein, in the classifying, the measurement sequence is classified as belonging to a group in which the similarity between the representative sequence and the measurement sequence is equal to or higher than a threshold value or a group in which the similarity between the representative sequence and the measurement sequence is highest.

9. The classification method according to Claim 1, wherein, in the measuring, a base sequence of a nucleic acid molecule in the measurement sample is measured as a measurement sequence using next-generation sequencing.

10. A classification device, comprising a processor, wherein the processor is configured to classify a measurement sequence, which has been measured, according to a plurality of groups into each of which base sequences of a plurality of nucleic acid molecules are grouped according to a specific rule, based on a similarity between a representative sequence and the measurement sequence, each of the representative sequences being set as a base sequence representing one of the respective groups.

11. A classification system comprising:

a measurement unit that measures a base sequence of a nucleic acid molecule in a measurement sample as a measurement sequence; and
a classifier that classifies the measurement sequence according to a plurality of groups into each of which base sequences of a plurality of nucleic acid molecules are grouped according to a specific rule, based on a similarity between a representative sequence and the measurement sequence that has been measured in the measurement unit, each of the representative sequences being set as a base sequence representing one of the respective groups.

12. A classification program executable by a computer to perform classification processing comprising classifying a measurement sequence, which has been measured, according to a plurality of groups into each of which base sequences of a plurality of nucleic acid molecules are grouped according to a specific rule, based on a similarity between a representative sequence and the measurement sequence, each of the representative sequences being set as a base sequence representing one of the respective groups.

13. A non-transitory recording medium storing a classification program, the classification program being executable by a computer to perform classification processing comprising classifying a measurement sequence, which has been measured, according to a plurality of groups into each of which base sequences of a plurality of nucleic acid molecules are grouped according to a specific rule, based on a similarity between a representative sequence and the measurement sequence, each of the representative sequences being set as a base sequence representing one of the respective groups.

# FIG.1

10

PREPARATION STEP ～11

↓

MEASUREMENT STEP ～12

↓

CLASSIFICATION STEP ～13

↓

SUBCLASSIFICATION STEP ～14

↓

COUNTING STEP ～15

↓

DETERMINATION STEP ～16

# FIG.2

MEASUREMENT DEVICE — 21

30

CPU — 31

ROM — 32

RAM — 33

STORAGE — 34

INPUT UNIT — 35

DISPLAY UNIT — 36

COMMUNICATION I/F — 37

39

20

# FIG.3

30

| CLASSIFICATION FUNCTION UNIT | 150 |
| SUBCLASSIFIER | 160 |
| COUNTING UNIT | 170 |
| DETERMINATION UNIT | 180 |

FIG.4

| | | | | | | |
|---|---|---|---|---|---|---|
| hsa-miR-570-3p | hsa-miR-548a-3p | hsa-miR-603 | hsa-miR-548d-3p | hsa-miR-548c-3p | hsa-miR-548g-5p | hsa-miR-548m |
| hsa-miR-548o-3p | hsa-miR-548f-5p | hsa-miR-548f-3p | hsa-miR-548h-3p | hsa-miR-548t-5p | hsa-miR-548u | hsa-miR-548x-5p |
| hsa-miR-548x-3p | hsa-miR-548z | hsa-miR-548ad-5p | hsa-miR-548ae-3p | hsa-miR-548ae-5p | hsa-miR-548ah-5p | hsa-miR-548ah-3p |
| hsa-miR-548aj-3p | hsa-miR-548aj-5p | hsa-miR-548am-3p | hsa-miR-548an | hsa-miR-548ap-5p | hsa-miR-548ap-3p | hsa-miR-548aq-3p |
| hsa-miR-548av-5p | hsa-miR-548av-3p | hsa-miR-548ay-3p | hsa-miR-548az-3p | hsa-miR-548bb-3p | hsa-miR-548bc | hsa-miR-320a-3p |
| hsa-miR-320b | hsa-miR-320c | hsa-miR-320d | hsa-miR-320e | hsa-miR-4429 | hsa-miR-17-5p | hsa-miR-20a-5p |
| hsa-miR-106a-5p | hsa-miR-106b-5p | hsa-miR-20b-5p | hsa-let-7b-5p | hsa-let-7g-5p | hsa-let-7i-5p | hsa-miR-4500 |
| hsa-miR-92a-3p | hsa-miR-92b-3p | hsa-miR-4279 | hsa-let-7b-3p | hsa-miR-98-3p | hsa-miR-16-5p | hsa-miR-195-5p |

## FIG.5

| GROUP | REPRESENTATIVE SEQUENCE | MOLECULAR NAME OF REPRESENTATIVE SEQUENCE | NUMBER OF MICRO RNA | SIMILAR SEQUENCE | | | | | | |
|---|---|---|---|---|---|---|---|---|---|---|
| 1 | CAAAAACCGCAAUUACUUUUGCA | hsa-miR-548h-3p | 34 | hsa-miR-570-3p | hsa-miR-548a-3p | hsa-miR-603 | hsa-miR-548d-3p | hsa-miR-548c-3p | hsa-miR-548g-5p | hsa-miR-548m |
| | | | | hsa-miR-548o-3p | hsa-miR-548f-5p | hsa-miR-548f-3p | hsa-miR-548h-3p | hsa-miR-548t-5p | hsa-miR-548u | hsa-miR-548x-5p |
| | | | | hsa-miR-548x-3p | hsa-miR-548z | hsa-miR-548ad-5p | hsa-miR-548ae-3p | hsa-miR-548ae-5p | hsa-miR-548ah-5p | hsa-miR-548ah-3p |
| | | | | hsa-miR-548aj-3p | hsa-miR-548aj-5p | hsa-miR-548am-3p | hsa-miR-548an | hsa-miR-548ap-5p | hsa-miR-548ap-3p | hsa-miR-548aq-3p |
| | | | | hsa-miR-548av-5p | hsa-miR-548av-3p | hsa-miR-548ay-3p | hsa-miR-548az-3p | hsa-miR-548bb-3p | hsa-miR-548bc | |
| 2 | AAAAGCUGGGUUGAGAGGGCGA | hsa-miR-320a-3p | 6 | hsa-miR-320a-3p | hsa-miR-320b | hsa-miR-320c | hsa-miR-320d | hsa-miR-320e | hsa-miR-4429 | |
| 3 | CAAAGUGCUUACAGUGCAGGUAG | hsa-miR-17-5p | 5 | hsa-miR-17-5p | hsa-miR-20a-5p | hsa-miR-106a-5p | hsa-miR-106b-5p | hsa-miR-20b-5p | | |
| 4 | UGAGGUAGUAGUUUGUGCUGUU | hsa-let-7i-5p | 4 | hsa-let-7b-5p | hsa-let-7g-5p | hsa-let-7i-5p | hsa-miR-4500 | | | |
| 5 | UAUUGCACUCGUCCCGGCCUCC | hsa-miR-92b-3p | 3 | hsa-miR-92a-3p | hsa-miR-92b-3p | hsa-miR-4279 | | | | |
| 6 | CUAUACAACUUACUACUUUCCC | hsa-miR-98-3p | 2 | hsa-let-7b-3p | hsa-miR-98-3p | | | | | |
| 7 | UAGCAGCACGUAAAUAUUGGCG | hsa-miR-16-5p | 2 | hsa-miR-16-5p | hsa-miR-195-5p | | | | | |

## INTERNATIONAL SEARCH REPORT

International application No.

**PCT/JP2024/018842**

| A. | CLASSIFICATION OF SUBJECT MATTER |
|---|---|

*G16B 30/00*(2019.01)i

FI:   G16B30/00

According to International Patent Classification (IPC) or to both national classification and IPC

| B. | FIELDS SEARCHED |
|---|---|

Minimum documentation searched (classification system followed by classification symbols)

G16B5/00-99/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | 並木洋平, 石田貴史, 秋山泰, 最長共通部分列に基づくDNA配列の高速クラスタリング, 情報処理学会研究報告 平成２２年度（５）［ＣＤ－ＲＯＭ］, 14 March 2011 (accepted), pp. 1-7, ISSN 1884-0930, (NAMIKI, Youhei et al., Fast DNA clustering based on Longest Common Subsequence, IPSJ SIG Technical Report, 2010-2011 (5), CD-ROM), 特に「２．２　本研究のDNA配列クラスタリング処理の流れ」,「６．結論」, non-official translation (in particular "2.2 Flow of DNA sequence clustering in this study", "6. Conclusion") | 1, 5-13 |
| Y | | 2, 4 |
| A | | 3 |
| Y | US 2015/0315641 A1 (SIEMENS AG) 05 November 2015 (2015-11-05) paragraphs [0049]-[0051] | 2, 4 |
| A | JP 2016-028590 A (NIPPON SOFTWARE MAN KK) 03 March 2016 (2016-03-03) entire text | 1-13 |

| ✓ | Further documents are listed in the continuation of Box C. | ✓ | See patent family annex. |
|---|---|---|---|

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 June 2024** | **02 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

International application No.

**PCT/JP2024/018842**

### C.    DOCUMENTS CONSIDERED TO BE RELEVANT

| Category* | Citation of document, with indication, where appropriate, of the relevant passages | Relevant to claim No. |
|-----------|------------------------------------------------------------------------------------|----------------------|
| A | WO 2014/126213 A1 (NEC SOLUTION INNOVATORS LTD.) 21 August 2014 (2014-08-21) entire text | 1-13 |

Form PCT/ISA/210 (second sheet) (July 2022)

**INTERNATIONAL SEARCH REPORT**

International application No.

**PCT/JP2024/018842**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

## INTERNATIONAL SEARCH REPORT
### Information on patent family members

International application No.

**PCT/JP2024/018842**

| Patent document cited in search report | | | Publication date (day/month/year) | Patent family member(s) | | | Publication date (day/month/year) |
|---|---|---|---|---|---|---|---|
| US | 2015/0315641 | A1 | 05 November 2015 | US | 2016/0273040 | A1 | |
| | | | | US | 2018/0340228 | A1 | |
| | | | | US | 2015/0292013 | A1 | |
| | | | | WO | 2014/075822 | A1 | |
| | | | | WO | 2014/075911 | A1 | |
| | | | | WO | 2014/075939 | A1 | |
| | | | | EP | 2733219 | A1 | |
| | | | | EP | 2733220 | A1 | |
| | | | | CN | 104903468 | A | |
| | | | | CN | 104981548 | A | |
| | | | | CN | 104968802 | A | |
| JP | 2016-028590 | A | 03 March 2016 | (Family: none) | | | |
| WO | 2014/126213 | A1 | 21 August 2014 | US | 2015/0379197 | A1 | |
| | | | | entire text | | | |
| | | | | EP | 2958038 | A1 | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023108941 A

**Non-patent literature cited in the description**

- **JIN**. Evaluation of Tumor-Derived Exosomal miRNA as Potential Diagnostic Biomarkers for Early-Stage Non-Small Cell Lung Cancer Using Next-Generation Sequencing. *Clinical Cancer Research*, 2017

- **ASAKURA**. A miRNA-based diagnostic model predicts resectable lung cancer in humans with high accuracy. *Communications Biology*, 2020